# EUROPEAN PATENT APPLICATION

(11) **EP 1 923 006 A2**
(43) Date of publication of application: **21.05.2008**
(21) Application number: 08075169.6
(22) Date of filing: 29.09.2005
(51) Int. Cl.: A61B 17/02, A61B 19/08

(54) **Disposable padding for a self-retaining retraction device**

(30) Priority: 30.09.2004 US 955457
(62) Divisional of application: 05256119.8
(71) Applicant: Codman & Shurtleff, Inc., Raynham, Massachusetts 02767 (US)
(72) Inventor: Sommerich, Bob, Norton Massachusetts 02766 (US); Bookwalter, John, Brattleboro VT, 05301 (US)
(74) Representative: Mercer, Christopher Paul

(57) **Abstract**

A disposable cover for a retractor of a self-retaining retractor system has an elastic sheath that can be easily placed and removed by elastically engaging the blade and a disposable pad attached on the elastic sheath. The elastic sheath can be an open cylinder (band shaped) or closed at one end (sock shaped) and is sized to cover a majority of the different size and shaped retractor blades. The elastic sheath retains the disposable pad on the blade by elastic compressive force. A single elastic sheath must be elastic enough to accommodate multiple sized blades and retain them in a fixed position while the blade is approximately vertically disposed within patient. The elastic sheath can be made from latex, nylon or any other surgical grade elastic or elastic type product.

## Description

### BACKGROUND OF THE INVENTION

### 1. Field of the Invention

This invention relates to a disposable retractor cover that selectively adheres to a retractor.

### 2. Discussion of the Related Art

In surgical operations on the chest or abdomen, it is customary to employ a retraction apparatus. Such a self-retaining retractor system is shown in U.S. Patent No. 4,254,763, which is assigned to the assignee of the present patent application, and whose specification is incorporated herein by reference. The surgical retractor assembly of that patent includes a support post which attaches directly to the surgical operating table. An extension arm may be attached to the support post for supporting an oval or round ring about the surgical incision. One or more retractor blades may be attached to the ring by means of the retractor ratchet mechanisms. Retractor blades of different sizes and shapes may be used to obtain the desired positioning and retraction of internal organs so that the operative site may be more completely exposed for the surgeon. Other retaining devices are the Weinstein retractor device and the O'Sullivan-O'Connor self-retaining abdominal retractor.

A retractor system can exert a great amount of force on the wound edges and on the tissues contacting those edges. As tissue is displaced, it is forced against the stiff retractor and often hard structures, such as bone. Delicate tissues, such as blood vessels and nerves, may experience trauma due to the force and duration of the tissue retraction. Therefore, from time to time, at the surgeon's discretion, the retractor should be released for short periods of time or the blades should be shifted to relieve the pressure on any one point. Releasing a retractor involves disengaging the attachment mechanism that holds the retractor in place and loosening and/or shifting the position of the retractor. This is a manual process and can be time consuming. Additionally, if the surgeon is involved in a long and delicate portion of the operation, the retractors may not be disengaged for a significant amount of time.

Further, it is common for stockinettes or surgical sponges to be placed between the tissue and retractor blade. These loose woven cloth sponges are often held in place by a metal retractor blade of sufficient width and depth. However the sponges can fall into the surgical site if the retractor is released. Additionally, tissue has a tendency to dry out during surgery due exposure to the operating room lighting and low humidity air. Sponges and stockinettes are absorbent and can draw needed moisture from the tissue and can increase the drying of the tissue.

Numerous prior art devices provide a form of padding for a retractor, but all have their limitations. U.S. Patent No. 5,709,646 to Lange discloses surgical retractor covers that are manufactured "inside-out," that is, the side that contacts the retractor is exposed prior to use. The exposed side is fully coated in adhesive and is "rolled-up" over the retractor so the adhesive side contacts the retractor as the cover is rolled up. It is a time consuming process to expose and "roll-up" each cover over the retractor. Lange's covers are also difficult to remove as they need to be "rolled-down" against the force of the adhesive, requiring longer clean-up times after a surgery. Further, Lange's covers extend over the entire retractor blade, which is typically not necessary since only one side of a retractor blade actually contacts the patent's tissue.

U.S. Patent Nos. 6,312,377 and D465,025 to Segermark et al. (collectively "Segermark") discloses tissue compression shields with removable inner pads. The shields and pads are actually placed horizontally inside a patient (i.e. parallel to the surgical table) because they are used for specific surgeries, for example, thoracic. The pads are not universal and must be used with a custom shell. The pad is typically injection molded to the shell and adhesive is not required. Since the pads are specific to the shell and the shell is specifically shaped for specific surgical techniques, Segermark pads cannot be used with any other type of retractor.

Removable covers are also used with speculum blades. U.S. Patent No. 5,007,409 to Pope and U.S. Patent Nos.4,807,600 and 4,492,220, both to Hayes (collectively "Hayes") discloses speculum blades that are inserted into the patient horizontally. However, neither Pope nor Hayes disclose how the covers are retained on the blades. An adhesive is not disclosed or required due to the placement of the blades in the patient. Further, both disclose that the covers should be used during an examination and should be easy to place and remove in order to facilitate the examination of numerous patients without significant delay. Use of an adhesive is not necessary and would be a time consuming step for the placement and removal of speculum covers. Additionally, a speculum is a specialized apparatus and the covers are unique to the shape of the blades of a speculum, and cannot be used by other types of retracting blades.

Another type of padding is disclosed in U.S. Patent No. 5,545,122 to Spruill. Spruill discloses padding in the form of an inflatable speculum wherein retraction pressure is applied by two bladders permanently fixed to the blades and inflated with compressed gas. The bladders are part of a specialized speculum. Further, the bladders are inflated, the examination or procedure is performed, and then the bladders are deflated. Thus, the bladders remain inflated during the examination and are not inflated and deflated throughout the course of the examination to relieve the retracting pressure from the tissue.

Thus, there is a need in the art for disposable, removable pads for retractors that can fit many different types of retractor blades. Further there is a need to allow the retracting force applied against the tissue to be temporally relieved during a surgery or procedure.

### SUMMARY OF INVENTION

A disposable cover for a retractor of a self-retaining retractor system has a handle and a blade disposed approximately vertically inside patient. The disposable cover has an elastic sheath that can be easily placed and removed by elastically engaging the blade and a disposable pad attached on the elastic sheath. The elastic sheath can be an open cylinder (band shaped) or closed at one end (sock shaped) and is sized to cover a majority of the different size and shaped retractor blades. The blades can be, for example, a Harrington, a Kelly, a Balfour, or a Gelpi blade and can be sized, for example, between 1 ½" to 3" wide and 2" to 6" long. Further there are standard flexible retractors with paddle shaped blades. The elastic sheath retains the disposable pad on the blade by elastic compressive force. A single elastic sheath must be elastic enough to accommodate multiple sized blades and retain them in a fixed position while the blade is approximately vertically disposed within patient. The elastic sheath can be made from latex, nylon or any other surgical grade elastic or elastic type product.

In addition, the elastic sheath can also include an adhesive layer intermittently disposed on the elastic sheath. The adhesive layer is not disposed along the entire elastic sheath. The adhesive layer can be disposed at an end closest to the handle, an end closest to a tip of the blade or both. The adhesive is selected to firmly engage the blade and/or handle and further be easily removed once the surgical procedure has been completed. This allows only the ends of the elastic sheath to engage the blade and handle. This expedites the placement and the removal of disposable cover and subsequent cleaning of the retractor while adding additional ways to secure the cover to the retractor. Additionally, the adhesive layer combined with the elastic will ensure proper and continuous placement throughout the surgery and still allow the disposable cover to be easily removed for subsequent disposal.

The adhesive layer can be a coating and/or a tape and must securely affix the elastic sheath to retractor, withstand possible repositioning of the blade within the patient, the fluids associated with a range of surgical procedures and be removable from the retractor with no or a minimal amount of residue remaining on the retractor to facilitate cleaning of the retractor. The coating for the adhesive layer can be, for example, at least one of Dow-Corning™ BIO-PSA^{®} 4500 and 4600 adhesive solids. A tape for the adhesive layer 106 can be, for example, 3M™ 9877, 1509, 1522, 1524 and 1577 double coated medical grade tapes.

The disposable pad is typically designed for a single use and is disposable after use. The disposable pad is attached to at least a portion of one side of the elastic sheath and can be designed to cover an entirety or majority of one side of the blade. The pad can also be designed to wrap around either the sides or the tip of the blade for additional protection. The disposable pad is malleable to form around the sides and tip but has some rigidity to retain its shape. Further, disposable pad can be compressible and deformable to provide protection to the retracted tissue, bone and organ. The disposable pad, in one embodiment, can be a nonabsorbent material to help prevent the retracted tissue from drying out. The disposable pad can be made of least one of a conformable polymeric material, a foam, a silastic, acrylic microspheres and a silicone based material.

In another embodiment, the disposable cover includes a disposable pad having an adhesive side and an adhesive layer is not disposed over the adhesive side. The adhesive layer affixes the disposable pad to the blade and can be removed easily after the surgical procedure is completed. The adhesive layer can be placed along the perimeter, in the center, or placed in a random or deliberate pattern on the disposable pad. The placement of the adhesive layer allows for quick placement and removal of the disposable pad without excessive adhesive to either place or peel from retractor.

The disposable cover can only adhere to one side or a portion of one side of the blade. Also, the disposable cover can be wrapped around the sides or tip to adhere to a portion of the opposite side of the blade.

The disposable cover can be individual adhesive disposable pads of common shapes and sizes or can be in a long length (e.g. a roll or sheet) and the correct length can be selected and cut to size by the surgical staff either before or after the incision has been made. Additionally, the disposable cover can have pre-perforated sections to simplify length selection. This removes the step of trimming the disposable cover to length.

Another embodiment of the disposable cover has the disposable pad and a bladder within the disposable pad that is elastically expandable under a pressure. A pump is in fluid communication, via a hose or line, with the bladder to supply the pressure using a compressed gas, a standard fluid or a sterile fluid. The bladder, when pressurized, acts as additional padding between the blade and the patient.

The bladder can be permanently disposed within the disposable pad, making the disposable cover fully disposable or the bladder can be removed from within the disposable pad so only the pad is disposed of. The removable bladder can enter the disposable pad through a preformed slit and removed once the surgical procedure is complete. The slit is sized to receive the bladder in the deflated condition and once the bladder is inflated it cannot be removed.

A bladder can also include one or more sub-bladders that are divisions of the bladder and can be individually inflated and deflated. The sub-bladders are in fluid communication with a pump with a single hose divided into a plurality of flow paths or an individual hose for each sub-bladder. The configuration allows one sub-bladder to deflate while another remains inflated. Thus, a portion of the same tissue remains retracted and another portion of tissue is relieved of the retraction pressure. The sub-bladders can be inflated and deflated in the cycles described below in relation to pump.

A pump is included in the system, linked to one or more bladders and/or sub-bladders, and can be a manual unit similar to a blood pressure cuff or can be actuated using a foot pedal. A manual system could be used for small surgeries, in rural areas where a powered pump may not be warranted and as a backup to a powered system.

A powered pump can be a simple device to pressurize and maintain a pressure in the bladder by controlling the pressurization and depressurization cycle. The pump can pressurize and depressurize the bladder and/or sub-bladders according to a set interval. A surgeon can determine a set time, for example 5 minutes, 20 minutes, 40 minutes, at which the bladder and/or sub-bladders deflate and an interval for the duration of the deflation. The pump can also be set to randomly pressurize and depressurize the bladder and/or sub-bladders. The pump can randomly pick an interval from a range of intervals and duration from a range of durations to pressurize and depressurize the bladder and/or sub-bladders.

Further, the pump can control numerous bladders and/or sub-bladders individually or in groups and can do so using multiple ports or electronically controlled valves. An additional parameter for numerous bladders can be an interval between bladders, groups of bladders, and/or sub-bladders. An example is that every bladder deflates and inflates concurrently, allowing for a simplified fluid communication system in which all of the bladders can be linked in parallel. However, surgeons may not want the entire surgical site to depressurize at once, shrinking the incised opening and then re-retracting the entire site.

Pump can be programmed to deflate and inflate bladders in series or in a chosen or random pattern or sequence. Pump can have multiple ports to control multiple bladders. Bladders can also be grouped together so multiple bladders react to one of the pump's ports.

Another embodiment includes an adhesive side and a bladder adhesive layer disposed on the adhesive side to removably affix the bladder to the blade. The bladder adhesive layer can be similar to the adhesive layer described above.

A further embodiment of the disposable cover has a fluid loop forming a thermal zone inside the disposable pad. A pump is in fluid communication, via a hose or line, with the fluid loop and pumps a fluid through the fluid loop. A temperature control device controls a temperature of the fluid. The temperature of the fluid directly correlates to the temperature of thermal zone. The fluid can be alternately heated or cooled in relation to the needs of the patient. The fluid loop can be disposable and disposed of with the disposable pad or can be removed from the disposable pad so only the pad is disposed.

Alternate embodiments include both the bladder and the fluid loop in fluid communication with each other and controlled from the same pump. The bladder adds a large reservoir of temperature controlled fluid to the thermal zone. This assists in accelerating the heating or cooling of the surrounding pad and tissue or organ.

Another embodiment includes an absorbent layer disposed within the disposable pad to retain a fluid. The fluid is typically a sterile fluid and moisturizes the absorbent layer. The disposable pad can be a partially absorbent material so as not to draw too much moisture from the tissue or organ the pad contacts. The absorbent layer then provides moisture to the disposable pad to transmit the moisture to the contacted tissue or organ. Both the material and/or the structure of the absorbent layer and disposable pad determine their individual absorbency.

In a further embodiment, the absorbent layer is filled with fluid prior to the placement of the disposable cover and the absorbent layer is designed to release the fluid over a period of time. The material of absorbent layer can dictate how fast the fluid will seep out into the disposable pad.

Alternately, a pump is placed in fluid communication, via tubes or hoses, with the absorbent layer and supplies the fluid to the absorbent layer. The pump can be manual or powered and can provide fluid at a low flow rate or over a set interval to provide the required amount of fluid to keep the absorbent layer and, thus the tissue, moisturized.

Additionally, a temperature control device can control a temperature of the fluid delivered to the absorbent layer. The fluid ultimately contacts the patient and the temperature of the fluid can be alternately heated or cooled in relation to the needs of the patient. Typically, the temperature of the fluid can be kept around body temperature to stay in equilibrium with the temperature of patient.

Another embodiment is a flexible, disposable retractor including a disposable pad with an adhesive side, a first end and a second end. An adhesive layer is disposed on the adhesive side of the disposable pad and is designed to adhere to skin, tissue and organ with enough strength to retract the tissue or the organ and anchor it against the patient's skin or the ring member. Further, the adhesive layer is gentle enough to be easily removed from the tissue or organ without any abrasion or residue. The first end adheres to the tissue or organ and the second end adheres to the patient, operating table, or ring member.

A cover layer covers the adhesive layer and is selectively removable to expose portions of the adhesive layer. The disposable retractor can come in various lengths and widths or on a roll for lengths to be chosen by the surgical staff. The adhesive layer typically covers the length of the disposable pad but the entire length of the adhesive layer does not need to be exposed.

The cover layer can be segmented and/or perforated to permit only a portion of the cover layer to be removed (or all of it, if required by the surgeon). The portion closest to first end can be exposed just enough to adhere to the organ that needs to be retracted. The surgeon then tensions disposable retractor to keep the organ in place and removes a portion of cover layer at the second end to adhere the second end to the patient, outside the incised area.

Additionally, a stiffening member can be included. The stiffening member can be a metal, alloy, or plastic and provides extra strength as the flexible, disposable retractor applies force to the tissue or organ. The stiffening member can be flexible and have no shape memory to allow the surgeon to bend the stiffening member into multiple positions. Alternately, the stiffening member is flexible but also has a shape memory and retains an approximately flat shape for a straight shaped disposable retainer.

If the stiffening member is added to the flexible, disposable retractor, the stiffening member can be scored to allow for predetermined bending points or to allow the stiffening member to be easily broken, allowing for a selection or reduction in length of the flexible, disposable retractor. Further, the disposable pad can have slits, slots, or holes along its edge. The slits provide extra flexibility to the disposable pad as the retractor is flexed and bent.

The present invention discloses numerous embodiments that require lines or hoses to be run from retractor to one or more pumps. The bladder, fluid loop, and the adsorbent layer can be used with an existing self-retaining retraction system.

An example of using the present invention with an existing Bookwalter™ system includes disposing a small grommet on a bottom side of pawl mechanism. The grommet has a base with an eyelet through which hose, can pass and be supported. The base can have an adhesive layer to either permanently or removably affix the grommet to the pawl mechanism. The adhesive can be a coating or tape and can be similar to adhesive layer.

Another embodiment of grommet includes left and right tension arms instead of an eyelet. The tension arms can be displaced to insert the hose between them and then the tension arms return to their original position and support the hose.

Further grommets can be disposed under the same or other pawl mechanisms engaging retractors, or pawl mechanism can be placed without a retractor to suspend the hose above the surgical field and direct it toward the pump. Additional grommets can be placed on elongated support post, elongate extension rod, coupling device, and ring member to further guide the hose to the pump.

In one embodiment, grommets with the tension arm embodiment are permanently affixed to the system. The tension arms can be sized to retain multiple hoses and the hoses can be removed if the bladder, fluid loop, and/or adsorbent layer are disposable. The grommets with an eyelet can be used if the hoses and the bladder, fluid loop, and/or adsorbent layer are permanent.

Alternately, for the disposable bladder, fluid loop, and/or adsorbent layer, the hoses can be packaged with numerous grommets already disposed on the hose. The grommets can slide along the hose and be removably affixed where needed. Once the procedure is complete, the grommets can be removed and disposed with the bladder, fluid loop, or adsorbent layer and the hose.

An advancement for a new Bookwalter™ system is a retractor with a channel formed in a handle. The hose can be disposed within channel to direct the hoses outside the perimeter of ring member. A handle with channel can be designed to fit existing the pawl mechanism or designed for a new, larger pawl mechanism.

The channel can run the length of handle or can be shorter. Typically, the channel is disposed on the "bottom" of the handle because the hose extends up from the tissue side of blade.

Alternately, the handle can have proximal and distal valves or hose connectors. The hose can be fluidly connected to distal connector and another hose or line can fluidly connect to the proximal connector. The hose can fluidly connect to the one or more pumps and the fluid flows through the channel. This embodiment allows the hose to be permanently attached to self-retaining retraction system while allowing the bladder, fluid loop, and/or adsorbent layer to be disposable. Further, the hose provided with the bladder, fluid loop, and adsorbent layer can be much shorter. Additionally, this simplifies setup and breakdown of the surgical site by the surgical staff.

Alternately, a ring member can have a hose raceway disposed on a bottom of the ring member. The hose raceway can have an opening smaller than the size of hose raceway to help retain the hose, but still allows the hose to pass into and out of the hose raceway. Additionally, either existing pawl mechanisms or a modified pawl mechanism can be used to engage ring member.

In an embodiment of the ring member, the opening opens to the inside of the ring member. This embodiment can be used if the hose comes directly from the retractor and not disposed within hose channel of retractor.

Alternately, the opening can open to the outside of the ring member. This embodiment can be used if the hose is disposed within the hose channel of retractor, exit the hose channel and enter hose raceway or if hose enters hose raceways directly.

Regardless of the system, all hoses terminate at one or more pumps for their respective purposes. Pumps are common the medical industry to pressurize air or liquid. The pumps can be controlled by a panel that allows a member of the surgical staff to set and change the parameters for the numerous embodiments, for example, temperature of the fluid loop and moisturizing fluid, the amount of pressurization of the bladders and sub-bladders, the deflation, reinflation interval between the bladders and selection of a particular bladder or sub-bladder. Further, these commands can be input by the surgeon during the surgery by either a hand unit or foot pedals. Both control systems are known in the art.

A method of retracting at least one of tissue, a bone and an organ of a patient using a retractor include the steps of inflating a bladder disposed on the blade. The bladder can be disposed on the blade either with bladder adhesive layer or disposed in a disposable pad. A member of the surgical team sets the blade and the bladder to apply a pressure against the tissue, bone or organ to retract it. The retractor can then be set into place, for example by a pawl mechanism on ring member.

In one embodiment of a Bookwalter™ system, the handle is inserted into the pawl mechanism after the blade has been set and locked into place. Once the retractors are set, the bladder can be deflated to relieve the retracting pressure against the tissue. The bladder is then reinflated to reapply the pressure against the tissue. The deflating and the reinflating steps can be performed using different intervals or patterns. The bladder can be deflated and inflated at timed intervals or random intervals. Control of the operations may be handled by a programmed microprocessor.

Another embodiment of the above method uses multiple bladders or sub-bladders. The bladder is inflated and a second bladder, disposed on a second blade, is also inflated. The blade and bladder are set as above and similarly, the second blade and the second bladder are set to a retraction position. The second bladder is both deflated and reinflated similarly to the bladder above. Further, the deflating and reinflating steps of the second bladder can be coordinated with the deflating and reinflating steps of the bladder. The deflation and reinflation of the bladder and the second bladder can be coordinated so as to synchronize the deflating and the reinflating steps of the bladder and the second bladder. Additionally, the bladders can be alternately or sequentially deflated and the reinflated. The deflation and reinflation pattern can be chosen by the surgeon depending on the type and duration of the procedure.

A similar method can be utilized for sub-bladders set on one blade. Both the first and second sub-bladders are inflated, deflated and then reinflated. Further, the deflating and reinflating steps of the second sub-bladder can be coordinated with the deflating and reinflating steps of the first sub-bladder. The deflation and reinflation of the first and second sub-bladders can be coordinated so to synchronize the deflating and the reinflating steps of the sub-bladders. Additionally, the sub-bladders can be alternately or sequentially deflated and the reinflated. The deflation and reinflation pattern can be chosen by the surgeon depending on the type and duration of the procedure or pre-defined profiles can be stored in a memory of the pump.

Synchronized coordination between the bladders is a basic system and all of the bladders can be in a parallel fluid circuit. For alternating and sequencing the bladders, multiple ports need to be provided for each bladder or groups of bladder. Optionally, a single pump with electronically controlled valves can also be used to direct the fluid flow accordingly.

Another method of retracting tissue, bone and organ using a self-retaining retractor include inflating a bladder disposed on the blade and setting the blade and the bladder to apply retraction pressure. These steps are similar to the steps above. A fluid can be circulated through a fluid loop disposed on the blade to control a temperature of the fluid. Controlling the temperature can allow a surgeon to cool or heat the tissue and/or the organ in contact with the blade and/or fluid loop. This can allow the contacted and surrounding tissue to be cooled to reduce swelling or heated to maintain a patient's temperature.

A further retraction method includes disposing an absorbent layer on the blade of the retractor and setting the blade and the absorbent layer to a retraction position. This similar to setting the blade and bladder as described above. Fluid is provided to the absorbent layer to moisturize the absorbent layer.

Moisturizing the tissue or organ prevents it from drying out when removed from the moist interior of the patient and exposed to the lighting and low humidity air typical in an operating room. The fluid is typically a sterile fluid and can be continuously provided or intermittently provided to the absorbent layer. Also, the temperature of the fluid can be controlled to heat or cool the fluid or keep it at body temperature. The fluid can be provided by a manual or a powered pump. Additionally, the absorbent layer can be soaked in the fluid prior to disposing it on the blade and the absorbent layer can be made of a material that slowly seeps the liquid out at a known rate.

An additional method of retracting tissue, bone or organ using a flexible, disposable retractor includes selectively exposing a portion of an adhesive layer on a first end of a disposable pad. Adhering the first end to the tissue and retracting the tissue by applying tension to the flexible, disposable retractor. A second portion of the adhesive layer on a second end is selectively exposed. The selective exposure of the adhesive layer can be performed by removing a segmented portion of a cover layer to expose the adhesive layer. The cover layer can be removed in finite lengths. The surgeon determines the amount of adhesive required for the specific task and can peel back the cover layer to expose only the amount required. The second end is then adhered to the patient, operating table or to the ring member to maintain the retraction of the tissue. Once the procedure or portion of the procedure is completed the flexible disposable retractor can be removed at the first end and the second end from the tissue and the patient, respectively.

To assist the surgeon in using the flexible, disposable retractor, the surgeon is able to select a length of the disposable pad and separate the length of disposable pad from a longer length of the disposable pad.

### BRIEF DESCRIPTION OF THE DRAWING FIGURES

The above and still further objects, features and advantages of the present invention will become apparent upon consideration of the following detailed description of a specific embodiment thereof, especially when taken in conjunction with the accompanying drawings wherein like reference numerals in the various figures are utilized to designate like components, and wherein:
Figure 1 is a perspective view illustrating a prior art surgical retractor assembly attached to a surgical operating table and ready for use in a surgical operation on a patient;
Figure 2A is a perspective view of an embodiment of a disposable cover set on a retractor according to the present invention;
Figure 2B is a cross section view along view 2B of Figure 2A of another embodiment.
Figure 3A is a side view of another embodiment of the disposable cover set on a retractor;
Figure 3B is a tissue side view of the embodiment of Figure 3A along line 3B;
Figure 3C is a perspective view of an embodiment that wraps around the tip and sides of a retractor blade;
Figure 3D is a cross-sectional along line 3D of Figure 3C;
Figure 4A is a perspective and partially cut-away view of a further embodiment of the disposable cover with a bladder;
Figure 4B is a perspective view of a multi-chamber inflatable bladder embodiment;
Figure 4C is a side view of another inflatable bladder with an adhesive of the present invention;
Figure 5 is a cross-sectional view of further embodiment of the disposable cover with a bladder and a thermal zone;
Figure 6 is a perspective and partially cut-away view of another embodiment of the disposable cover with an absorbent layer;
Figure 7A is a side view of a flexible, disposable retractor of the present invention;
Figure 7B is a top view of the flexible, disposable retractor in use;
Figure 7C is a top view of another embodiment of the flexible, disposable retractor of the present invention;
Figure 8A is a perspective view of a self-retaining retractor system with grommets of the present invention;
Figure 8B is a magnified side view of a self-retaining retractor system with grommets
Figures 9A and 9B are perspective views of different grommets according to the present invention;
Figures 10A is a side and partially cut-away view of a retractor of the present invention;
Figure 10B is a proximal end view of the retractor of Figure 10A;
Figure 11A is a cross-sectional view of a ring member according to the present invention;
Figure 11B is a cross-sectional view of another embodiment of the ring member according to the present invention;
Figure 12 is a flow diagram for a method of inflating and deflating a bladder disposed on a blade according to the present invention;
Figure 13 is a flow diagram for a method of inflating and deflating multiple bladders according to the present invention;
Figure 14 is a flow diagram for a method of inflating and deflating sub-bladders according to the present invention;
Figure 15 is a flow diagram for a method of controlling a temperature of the retractor according to the present invention;
Figure 16 is a flow diagram for a method of providing a fluid to an absorbent layer according to the present invention; and
Figure 17 is a flow diagram for a method of using a flexible, disposable retractor according to the present invention.

### DETAILED DESCRIPTION OF THE PREFERRED EMBODIMENT

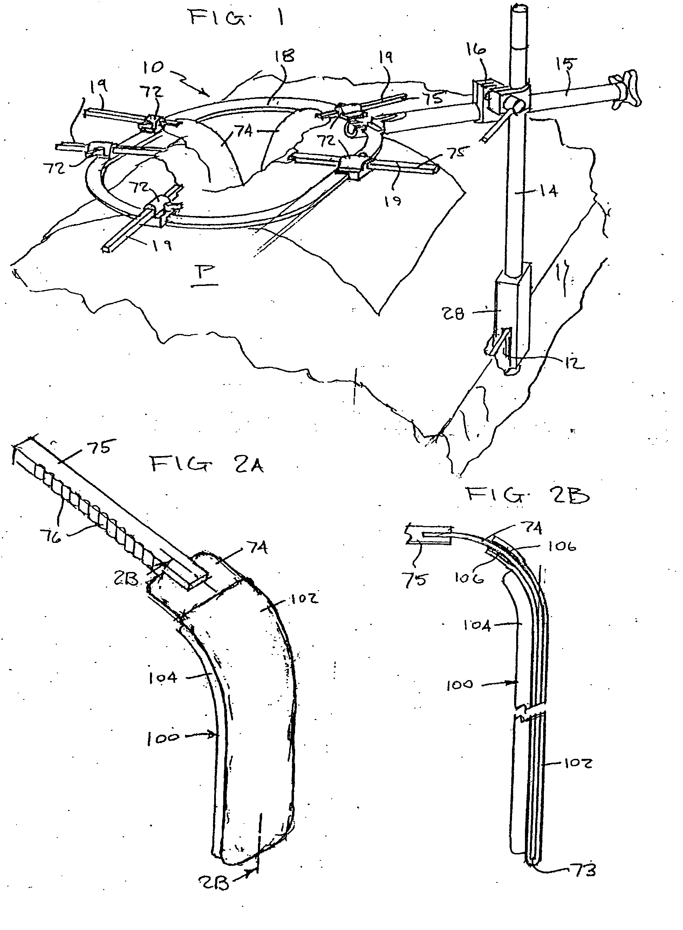

Referring to Figure 1, a Bookwalter™ self retaining retractor system is illustrated. A retractor assembly 10 attached to a surgical operating room table 12 and in a position on a patient P. The main components are an elongated support post 14, an elongate extension rod 15 which is connected to support post 14 by a coupling device 16, a substantially flat, oval-shaped ring member 18 adjustably affixed to extension rod 15 and a plurality of retractors 19 adjustably mounted on ring member 18. At the lower portion of support post 14 is an adjustable clamp assembly 28 whereby support post 14 can be attached to the surgical operating table 12.

Retractor 19 is mounted on ring member 18 with a quick-release pawl attachment mechanism 72. The retractor includes a blade 74 which is inserted into an incision in patient P for restraining tissue, organ, bone and the like during the surgical procedure. A handle 75 extends from blade 74. Along one surface of handle 75 there are a plurality of ratchet teeth 76 which are spaced to provide small incremental adjustments of the handle. Handle 75 is inserted through pawl mechanism 72 and which holds retractor 19 so that the handle 75 extends in a general radial direction and is thus adjustable in the radial direction.

It can be seen that each retractor 19 may be adjustably positioned to any desirable annular position on ring member 18. Also, each retractor 19 may be adjusted in a general radial direction toward or away from the open center of the ring member 18.

Figure 2A illustrates a retractor 19 having a handle 75 and a blade 74 disposed approximately vertically inside the patient. A disposable cover 100 for the retractor includes an elastic sheath 102 removably elastically engaging the blade and a disposable pad 104 disposed on the elastic sheath 102. Elastic sheath 102 can be an open cylinder (band shaped) or closed at one end (sock shaped). Elastic sheath 102 is sized to cover a majority of the different sized and shaped retractor blades 74. Blades 74 can be, for example, a Harrington, a Kelly, a Balfour, or a Gelpi blade (not illustrated) and can be sized, for example, between 1 ½" to 3" wide and 2" to 6" long. The elastic sheath 102 can retain disposable pad 104 on blade 74 by elastic compressive force. A single elastic sheath 102 must be elastic enough to accommodate multiple sized blades 74 and to retain them in a fixed position while blade 74 is approximately vertically disposed within patient P. Elastic sheath 102 can be made from latex, nylon or any other surgical grade elastic or elastic type product.

In addition Figure 2B illustrates the elastic sheath 102 of disposable cover 100 can also include an adhesive layer 106 disposed on the elastic sheath 102. Adhesive layer 106 can be located intermittently so that it is not disposed along the entire elastic sheath 102. An example, adhesive layer 106 can be disposed only at an end closest to the handle 75, an end of the blade 74 closest to a tip 73 of blade 74 or both. The adhesive is selected to be removably engagable to at least the blade 74 and/or the handle 75. This allows only the ends of the elastic sheath 102 to engage blade 74 and handle 74, which expedites the placement and removal of disposable cover 100 and the subsequent cleaning of the retractor 19, while adding additional ways to secure the cover 100 to the retractor 19. Additionally, the adhesive layer 106 combined with the elastic will ensure proper and continuous placement of disposable pad 104 on blade 74 throughout the surgery and still allow the disposable cover 100 to be easily removed for subsequent disposal.

The adhesive layer 106 can be a coating and/or a tape. The adhesive layer 106 must securely affix the elastic sheath 102 to retractor 19, withstand possible repositioning of the blade 74 within the patient P, withstand the fluids associated with a range of surgical procedures and be removable from retractor 19 with no or a minimal amount of residue remaining on the retractor 19 to facilitate cleaning of the retractor 19. The coating for the adhesive layer 106 can be, for example, at least one of Dow-Corning™ BIO-PSA^{®} 4500 and 4600 adhesive solids. A tape for the adhesive layer 106 can be, for example, 3M™ 9877, 1509, 1522, 1524 and 1577 double coated medical grade tapes.

Disposable pad 104 is typically designed for a single use and is disposable after use. The disposable pad 104 is attached to at least a portion of one side of the elastic sheath 102. The pad can be designed to cover an entirety or majority of one side of blade 74 and can also be designed to wrap around either the sides or the tip 73 of the blade 74 for additional protection. The disposable pad 104 can be malleable to form around the sides and tip 73 but may have some rigidity to retain its shape. Further, disposable pad 104 can be compressible and deformable to provide protection to the retracted tissue, bone and organ. The disposable pad 104, in one embodiment, can be a nonabsorbent material to help prevent the retracted tissue from drying out. The disposable pad 104 can be made of least one of a conformable polymeric material, a foam, a silastic, acrylic microspheres and a silicone based material.

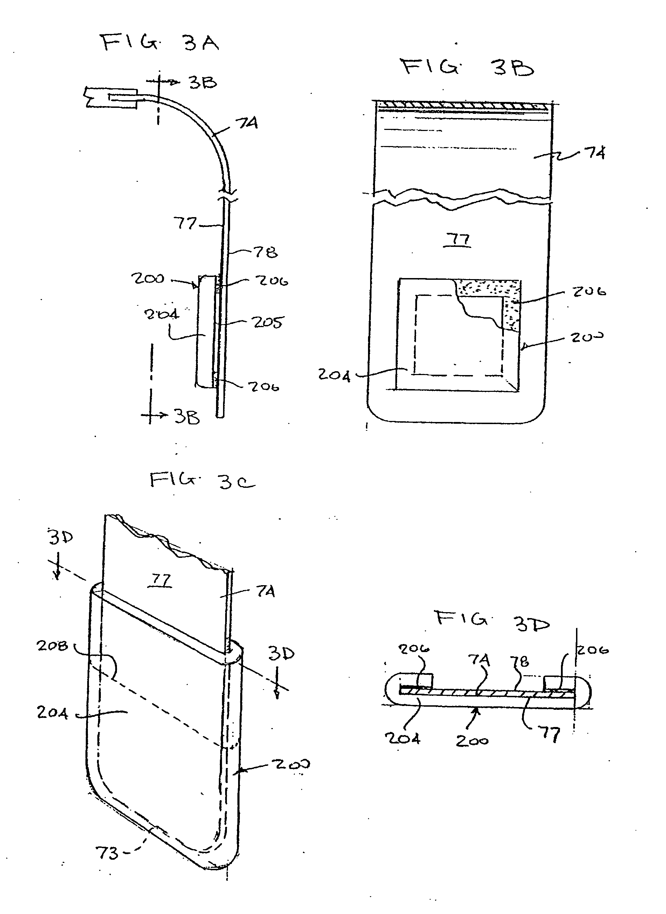

In another embodiment, illustrated in Figures 3A 3D, a disposable cover 200 for retractor 19 where blade 74 has a tissue side 77 that contacts at least one of the patient's tissue, bone and/or organ. Blade 74 also has an opposite side 78 of the tissue side 77. Disposable cover 200 includes a disposable pad 204 having an adhesive side 205 and an adhesive layer 206 intermittently disposed on the adhesive side 205.

Adhesive layer 206 adheres the disposable pad 204 to blade 74 and can be removed easily after the surgical procedure is completed. Figure 3A shows adhesive layer 206 is not disposed over the entire adhesive side 205 of disposable pad 204. The adhesive layer 206 can be placed along the perimeter, in the center, or placed in a random or deliberate pattern on the adhesive side 205 of disposable pad 204. The placement of the adhesive layer 206 allows for the quick placement and removal of the disposable pad 204 without excessive adhesive to either place or peel from retractor 19.

Figures 3A and 3B show an example of a disposable cover 200. A position of disposable cover 200 where the disposable pad 204 only adheres to the tissue side 77 or a portion of the tissue side 77 of blade 74. Figures 3C and 3D illustrate a disposable cover 200 which is wider than blade 74 so it can be further wrapped around the sides or tip 73 or blade 74 to adhere to a portion of the opposite side 78 of blade 74.

Disposable cover 200 can be individual adhesive disposable pads of common shapes and sizes or can be in a long length (e.g. a roll or sheet) and the correct length can be selected and cut to size by the surgical staff either before or after the incision has been made. Additionally, disposable cover 200 can have pre-perforated sections 208 to simplify length selection. This simplifies the step of trimming the disposable cover 200 to length, i.e. disposable cover 200 can be torn at preset locations 208 along its length.

As above, disposable pad 204 can have the same physical properties and be made of the same materials as disposable pad 104. Accordingly, adhesive layer 206 can be either a coating or a tape and have properties similar to adhesive layer 106.

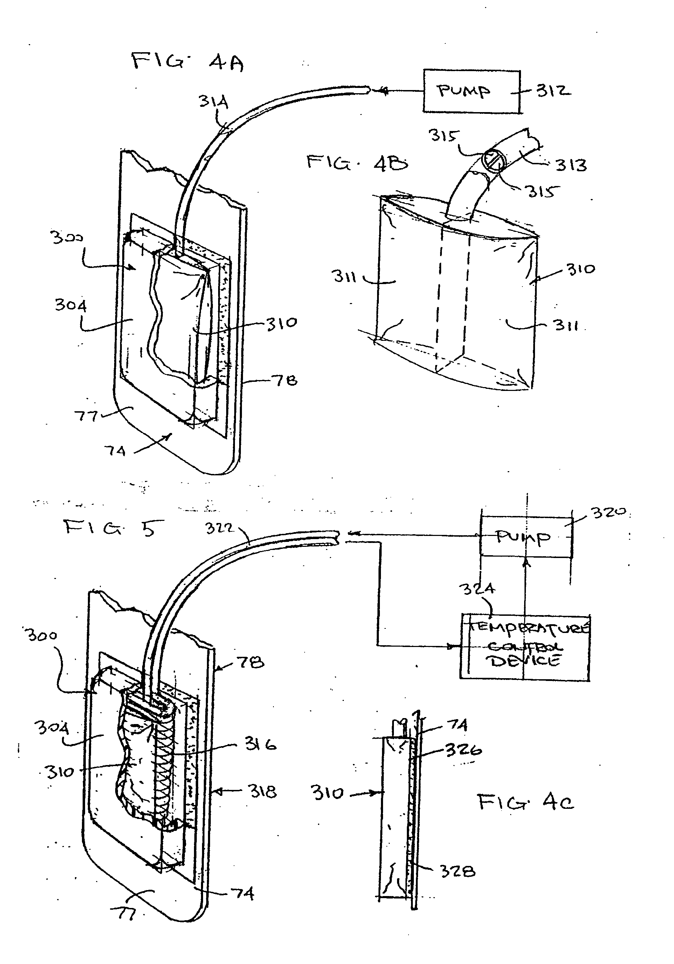

Figure 4A illustrates a disposable cover 300 having a disposable pad 304 removably disposed on retractor blade 74. Disposable pad 304 includes a bladder 310 disposed within the disposable pad 304, which bladder is elastically expandable under a pressure. A pump 312 is in fluid communication, via a hose or line 314, with bladder 310 to supply the pressure via compressed gas, a conventional fluid or a sterile fluid. Bladder 310, when pressurized, acts as additional padding between the blade 74 and the patient.

Disposable cover 300 can be designed so that bladder 310 is disposable and is permanently located within pad 304. This embodiment of disposable cover 300 is fully disposable and is designed for one use. Alternately, bladder 310 can be made removable from within pad 304 and only pad 304 is disposed. Bladder 310 can be inserted into disposable pad 304 through a preformed slit 314 and can be removed once the surgical procedure is complete. The slit 314 can be sized to receive bladder 310 only in the deflated condition so that once bladder 310 is inflated, it cannot be removed.

Figure 4B illustrates that bladder 310 can also include one or more sub-bladders 311. Sub-bladders 311 are divisions of bladder 310 and can be individually inflated and deflated. The sub-bladders 311 can be in fluid communication with pump 312 with a single hose 313 divided into a plurality of flow paths 315 or multiple hoses 314. Sub-bladders 311 permit one sub-bladder 311 to deflate while another remains inflated, allowing a portion of the same tissue to remain retracted and another portion of tissue to be relieved of the retraction pressure. Sub-bladders 311 can be inflated and deflated in the cycles described below in relation to pump 312.

Pump 312 can be a manual unit similar to a blood pressure cuff, and can be linked to one or more bladders 310 and/or sub-bladders 311. Additionally, the manual pump can be actuated using a foot pedal. A manual system could be used for small surgeries, in rural areas where a powered pump may not be warranted and as a backup to a powered system.

Powered pump 312 can be a simple device to pressurize and maintain a pressure in bladder 310. Pump 312 can also control a pressurization and depressurization cycle, i.e. pump 312 can pressurize and depressurize the bladder 310 and/or sub-bladders 311 according to a set interval. A surgeon can determine a set time, for example 5 minutes, 20 minutes, 40 minutes, at which the bladder 310 and/or sub-bladders 311 deflate and an interval for the duration of the deflation. Pump 312 can also be set to randomly pressurize and depressurize the bladder 310 and/or sub-bladders 311. Pump 312 randomly picks an interval from a range of intervals and duration from a range of durations to pressurize and depressurize bladder 310 and/or sub-bladders 311.

Pump 312 can control numerous bladders 310 and/or sub-bladders 311, individually or in groups, using multiple ports or electronically controlled valves. An additional parameter for numerous bladders 310 can be a time interval between bladders 310, groups of bladders 310, and/or sub-bladder 311. An example is that every bladder 310 deflates and inflates concurrently. This embodiment allows for a simplified fluid communication system, i.e., all of the bladders can be linked in parallel. However, surgeons may not want the entire surgical site to depressurize at once, shrinking the incised opening and then re-retracting the entire site.

Pump 312 can be programmed to deflate and inflate bladders 310 in series or in a chosen or random pattern or sequence. Pump 312 can have multiple ports to control multiple bladders 310. Bladders 310 can also be grouped together so multiple bladders 310 react to one of the pump's 312 ports.

Another embodiment, illustrated in Figure 4C, is bladder 310 which has an adhesive side 326 and a bladder adhesive layer 328 disposed on the adhesive side 326 to removably affix bladder 310 to blade 74. Bladder adhesive layer 328 can be similar to adhesive layer 106, 206.

Figure 5 illustrates a further embodiment of disposable cover 300. Disposable pad 304 can further receive a fluid loop 316 to form a thermal zone 318. A pump 320 is in fluid communication, via a hose or line 322, with the fluid loop 316 and pumps a fluid through the fluid loop 316. A temperature control device 324 controls a temperature of the fluid. The temperature of the fluid directly correlates to the temperature of thermal zone 318. Thus, the fluid can be alternately heated or cooled causing a heating and cooling of thermal zone 318 in relation to the needs of the patient.

Fluid loop 316 can be disposable and can be permanently located within the disposable pad 304. Alternately, the fluid loop 316 can be a more durable system and can be made removable from the disposable pad 304, so only pad, 304 is disposed.

Alternate embodiments include both the bladder 310 and fluid loop 316 in fluid communication with each other and controlled from the same pump 312, 320. Bladder 310 adds to thermal zone 318 a large reservoir of temperature controlled fluid. This assists in accelerating the heating or cooling of the surrounding pad and tissue or organ.

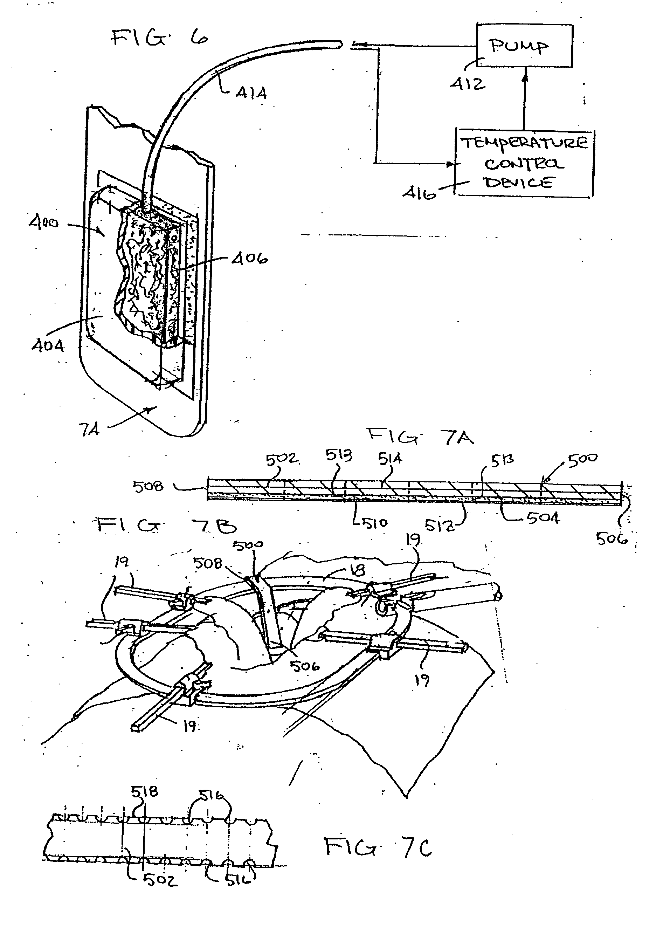

Figure 6 illustrates disposable cover 400 having a disposable pad 404 that can be removed from blade 74 and an absorbent layer 406 disposed within the disposable pad 404 to retain a fluid. The fluid is typically a sterile fluid and moisturizes the absorbent layer 406. Disposable pad 404 can be a partially absorbent material so as not to draw too much moisture from the tissue or organ of the patient P that is in contact with the pad. The absorbent layer 406 provides moisture to the disposable pad 404 which transmits the moisture to the contacted tissue or organ. Both the material and/or the structure of the absorbent layer 406 and disposable pad 404 determine their individual absorbency.

In one embodiment, the absorbent layer 406 is filled with fluid prior to the placement of cover 400, and absorbent layer 406 is designed to release the fluid over a period of time. The material of absorbent layer 406 dictates how fast the fluid will seep out into disposable pad 404.

Alternately, a pump 412 can be placed in fluid communication, via tubes or hoses 414 with the absorbent layer 406. Pump 412 supplies the fluid to the absorbent layer 406. Such a pump can be manual or powered, and can provide fluid at a low flow rate or over a set interval to provide the required amount of fluid to keep the absorbent layer 406, and hence the tissue, moisturized.

Additionally, a temperature control device 416 can control a temperature of the fluid delivered to the absorbent layer 406. The fluid ultimately contacts the patient and the temperature of the fluid can be alternately heated or cooled in relation to the needs of the patient P. Typically, the temperature of the fluid can be kept around body temperature to stay in equilibrium with the temperature of patient P.

Regarding to Figure 7A, it illustrates a flexible, disposable retractor 500 including a disposable pad 502 having an adhesive side 504, a first end 506 and a second end 508. An adhesive layer 510 is disposed on the adhesive side 504 of the disposable pad 502. As shown in Figure 7B, adhesive layer 510 is designed to adhere to skin, tissue and organ with enough strength to retract the tissue or the organ and anchor it against the patient's skin or ring member 18. Further, the adhesive layer 510 is gentle enough to be easily removed from the tissue or organ without any abrasion or residue. The first end 506 removably adheres to the tissue or organ and second end 508 removably adheres to the patient, operating table, or ring member 18.

A cover layer 512 covers the adhesive layer 510 and is selectively removable to expose portions of the adhesive layer 510. Disposable retractor 500 can come in various lengths and widths or on a roll for lengths to be chosen by the surgical staff. The adhesive layer 510 typically covers the length of the disposable pad 502 but the entire length of the adhesive layer 510 does not need to be exposed.

Cover layer 512 can be segmented and/or perforated 513 to permit only a portion of the cover layer 512 to be removed (or all of it, if required by the surgeon). The portion closest to first end 506 can be exposed just enough to adhere to the organ that needs to be retracted. The surgeon then tensions disposable retractor 500 to keep the organ in place and removes a portion of cover layer 512 at the second end 508 to adhere the second end to the patient, outside the incised area.

Additionally, a stiffening member 514, made of, for example, a metal, alloy or plastic, can be included in disposable pad 502. It can provide extra strength as the flexible, disposable retractor 500 applies force to the tissue or organ. The stiffening member 514 can be flexible and have no shape memory to allow the surgeon to bend the stiffening member 514 into multiple positions. Alternately, the stiffening member 514 is flexible but also can have a shape memory so it retains an approximately flat shape.

If stiffening member 514 is added to flexible, disposable retractor 500, the stiffening member 514 can be scored 513 to allow for predetermined bending points or to allow the stiffening member 514 to be easily broken to allow for a selection or reduction in length of the retractor 500. Further illustrated in Figure 7C, disposable pad 502 can have slits, slots, or holes 516 along its edge 518. Slits 516 provide extra flexibility to the disposable pad 502 as the retractor 500 is flexed and bent.

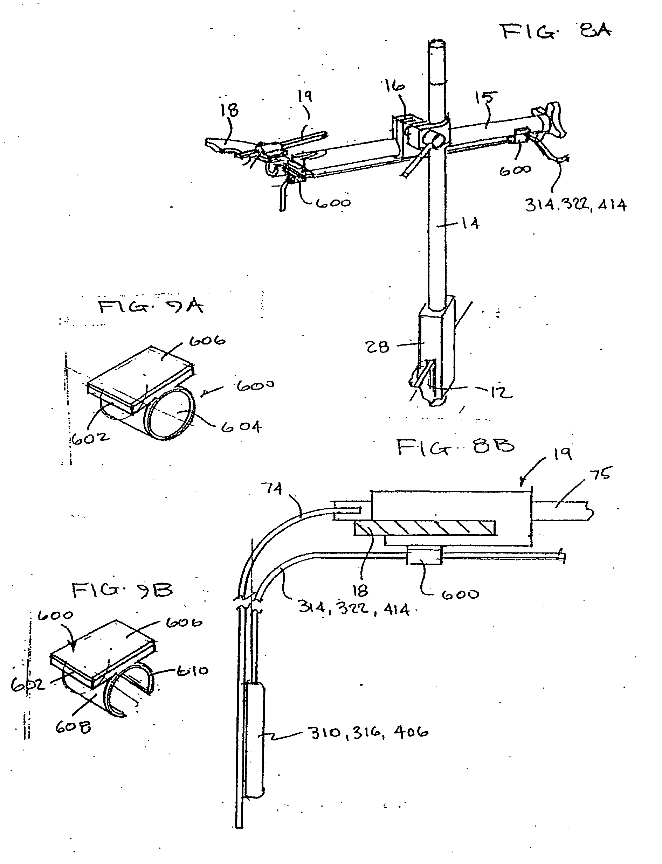

The present invention discloses numerous embodiments that require lines or hoses 314, 322, 414 to be run from retractor 19 to one or more pumps 312, 320, 412. The bladder 310, fluid loop 316, and the adsorbent layer 406 can be used with an existing self-retaining retraction system. Figures 8A and 8B illustrate one example of using the present invention with an existing Bookwalter™ system 10 (see Figure 1). Small grommet 600 can be disposed on a bottom side 71 of pawl mechanism 72. Figure 9A illustrates grommet 600 having a base 602 with an eyelet 604 through which hose 314, 322, 414 can pass and be supported. Base 602 can have an adhesive layer 606 to either permanently or removably affix the grommet 600 to pawl mechanism 72. The adhesive 606 can be a coating or tape and can be similar to adhesive layer 106.

Another embodiment of grommet 600 includes left and right tension arms 608, 610 instead of eyelet 604 and as illustrated in Figure 9B. The tension arms 608, 610 can be displaced to insert hose 314, 322, 414 between them and then tension arms 608, 610 return to their original position and support hose 314, 322, 414.

Further grommets 600 can be disposed under the same or other pawl mechanisms 72 engaging retractors 19, or pawl mechanism 72 can be placed without a retractor 19 to suspend hose 314, 322, 414 above the surgical field and direct it toward pump 312, 320, 412. Additional grommets 600 can be placed on elongated support post 14, elongate extension rod 15, coupling device 16, and ring member 18 to further guide hose 314, 322, 414 to pump 312, 320,412.

In one embodiment, grommets 600 with the tension arm embodiment are permanently affixed to the system. Tension arms 608, 610 can be sized to retain multiple hoses 314, 322, 414 and the hoses 314, 322, 414 can be removed if the bladder 310, fluid loop 316, and/or adsorbent layer 406 are disposable. The grommets 600 with eyelet 604 can be used if the hoses 314, 322, 414 and bladder 310, fluid loop 316, and/or adsorbent layer 406 are permanent.

Alternately, for the disposable bladder 310, fluid loop 316, and/or adsorbent layer 406, the hoses 314, 322, 414 can be packaged with numerous grommets 600 already disposed on the hose 314, 322, 414. The grommets 600 can slide along hose 314, 322, 414 and be removably affixed where needed. Once the procedure is complete, the grommets 600 can be removed and disposed with the bladder 310, fluid loop 316, or adsorbent layer 406, and hose 314, 322, 414.

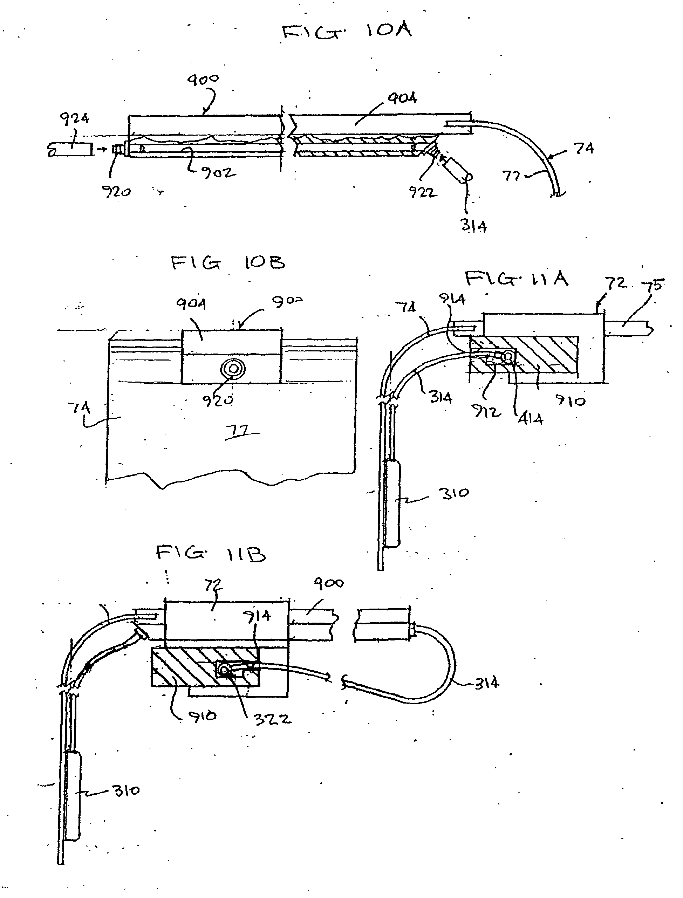

The above embodiment can be used with an existing Bookwalter™ system. Figures 10A, 10B, 11A and 11B illustrate advancements for new Bookwalter™ systems. Figures 10A and 10B illustrate retractor 900 with a channel 902 formed in a handle 904. Hose 314, 322, 414 can be disposed within channel 902 to direct the hoses outside the perimeter of ring member. Handle 904 with channel 902 can be designed to fit existing pawl mechanism 72 or designed for a new, larger pawl mechanism (not illustrated).

Channel 902 can run the length of handle 904 or can be shorter. Typically, the channel 902 is disposed on the "bottom" of the handle 904 because hose 314, 322, 414 extends up from the tissue side 77 of blade 74.

Alternately, handle 904 can have a proximal 920 and distal 922 valves or hose connectors. Hose 314, 322, 414 can be fluidly connected to distal connector 922 and another hose or line 924 can fluidly connect to the proximal connector 920. Hose 924 can fluidly connect to the one or more pumps 312, 320, 412 and the fluid flows through channel 902. This embodiment allows hose 924 to be permanently attached to self-retaining retraction system 10 while allowing bladder 310, fluid loop 316, and/or adsorbent layer 406 to be disposable. Further, the hose 314, 320, 412 provided with bladder 310, fluid loop 316, and adsorbent layer 406 can be much shorter. Additionally, this simplifies setup and breakdown of the surgical site by the surgical staff.

Alternately, as shown in Figure 11A and 11B, a ring member 910 can have a hose raceway 912 disposed on a bottom of the ring member 910. Hose raceway 912 can have an opening 914 smaller than the size of hose raceway 912 to help retain hose 314, 322, 414 but still allows hose 314, 322, 414 to pass into and out of the hose raceway 912. Additionally, either existing pawl mechanisms 72 or a modified pawl mechanism (not illustrated) can be used to engage ring member 910.

Figure 11A illustrates a cross section of ring member 910 where opening 914 opens to the inside of the ring member 910. This embodiment can be used if hose 314, 322, 414 come directly from the retractor 19 and not disposed within hose channel 902 of retractor 900.

Alternately, Figure 11B illustrates a cross section of ring member 910 where opening 914 opens to the outside of the ring member 910. This embodiment can be used if hose 314, 322, 414 are disposed within hose channel 902 of retractor 900, exit the hose channel 902 and enter hose raceway 912 or if hose 924 enters hose raceways 912 directly.

Regardless of the system, all hoses 314, 322, 414, 924 terminate at one or more pumps 312, 320, 412 for their respective purposes. Pumps 312, 320, 412 are common in the medical industry to pressurize air or liquid. The pumps 312, 320, 412 can be controlled by a panel that allows a member of the surgical staff to set and change the parameters for the numerous embodiments, for example, temperature of the fluid loop and moisturizing fluid, the amount of pressurization of the bladders and sub-bladders, the deflation, reinflation interval between the bladders and selection of a particular bladder or sub-bladder. Further, these commands can be input by the surgeon during the surgery by either a hand unit or foot pedals. Both control systems are known in the art.

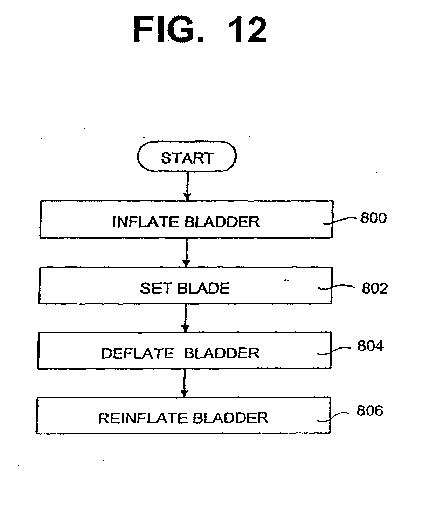

A method of retracting at least one of tissue, a bone and an organ of a patient using a retractor 19 is illustrated in Figure 12. The steps of the method include inflating a bladder disposed on the blade (step 800). The bladder can be disposed on the blade 74 either with bladder adhesive layer 328 or disposed in a disposable pad 304. A member of the surgical team sets the blade and the bladder to apply a pressure against the tissue, bone or organ to retract it (step 802). The retractor can then be set into place, for example by a pawl mechanism 72 on ring member 18. In one embodiment of a Bookwalter™ system, the handle 75 is inserted into the pawl mechanism 72 after the blade 74 has been set and locked into place (see Figure 1). Once the retractors are set, the bladder can be deflated to relieve the retracting pressure against the tissue (step 804). The bladder is then reinflated to reapply the pressure against the tissue (step 806). The deflating and the reinflating steps can be performed using different intervals or patterns. The bladder can be deflated and inflated at timed intervals or random intervals. Control of the operations may be handled by a programmed microprocessor.

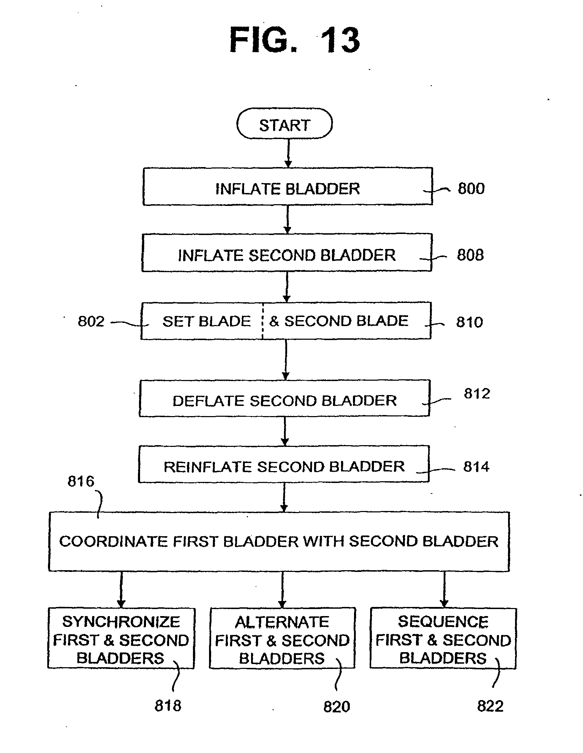

Figure 13 illustrates another embodiment of the above method using multiple bladders or sub-bladders. The bladder is inflated (step 800) and a second bladder, disposed on a second blade, is also inflated (step 808). The blade and bladder are set as above (step 802) and similarly, the second blade and the second bladder are set to a retraction position (step 810). The second bladder is both deflated (step 812) and reinflated (step 814) similarly to the bladder above. Further, the deflating and reinflating steps of the second bladder can be coordinated with the deflating and reinflating steps of the bladder (step 816). The deflation and reinflation of the bladder and the second bladder can be coordinated so as to synchronize the deflating and the reinflating steps of the bladder and the second bladder (step 818). Additionally, the bladders can be alternately (step 820) or sequentially (step 822) deflated and the reinflated. The deflation and reinflation pattern can be chosen by the surgeon depending on the type and duration of the procedure.

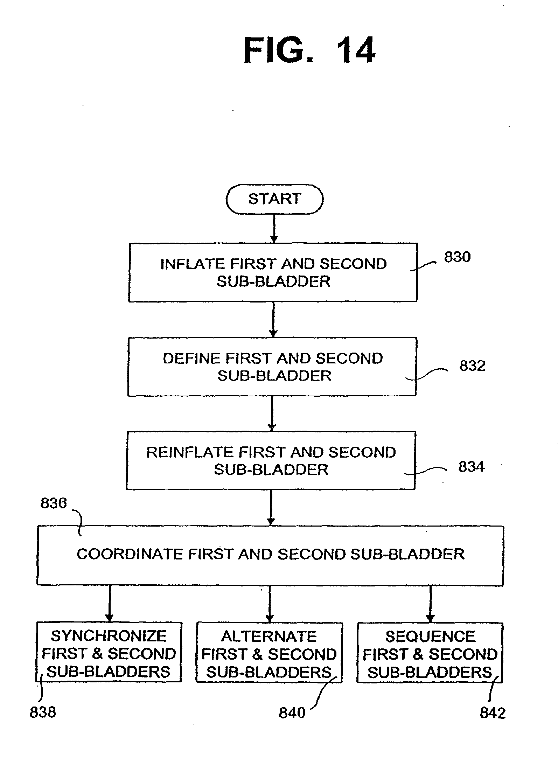

A similar method can be utilized for sub-bladders set on one blade. Figure 14 illustrates both first and second sub-bladders are inflated (step 830). First and second sub-bladders are deflated (step 832) and reinflated (step 834). Further, the deflating and reinflating steps of the second sub-bladder can be coordinated with the deflating and reinflating steps of the first sub-bladder (step 836). The deflation and reinflation of the first and second sub-bladders can be coordinated so to synchronize the deflating and the reinflating steps of the sub-bladders (step 838). Additionally, the sub-bladders can be alternately (step 840) or sequentially (step 842) deflated and the reinflated. The deflation and reinflation pattern can be chosen by the surgeon depending on the type and duration of the procedure or pre-defined profiles can be stored in a memory of the pump.

Synchronized coordination between the bladders is a basic system and all of the bladders can be in a parallel fluid circuit. For alternating and sequencing the bladders, multiple ports need to be provided for each bladder or groups of bladder. Optionally, a single pump with electronically controlled valves can also be used to direct the fluid flow accordingly.

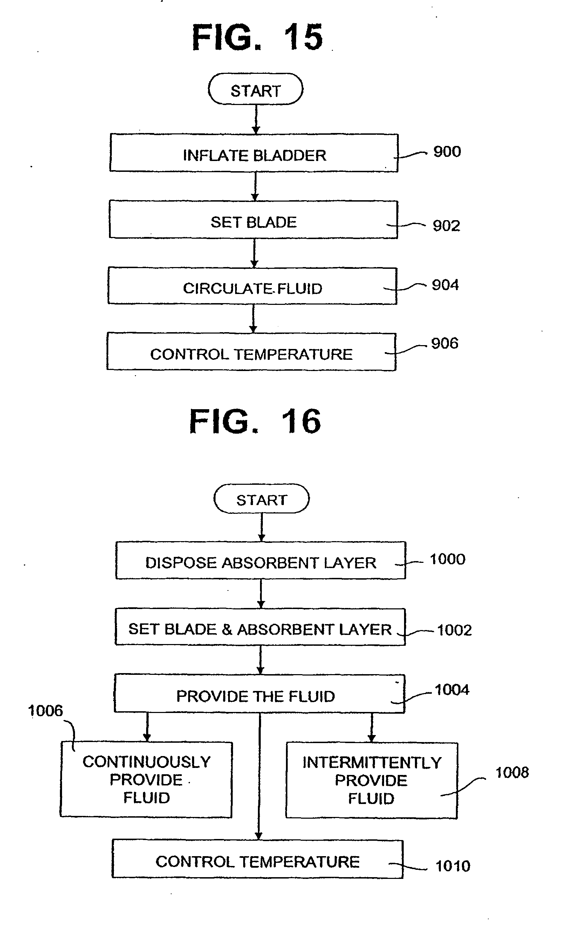

Figure 15 illustrates another method of retracting tissue, bone and organ using a self retaining retractor 19. The steps include inflating a bladder disposed on the blade (step 900) and setting the blade and the bladder to apply retraction pressure (step 902). These steps are similar to steps 800 and 802 above. A fluid can be circulated through a fluid loop disposed on the blade (step 904) to control a temperature of the fluid (step 906). Controlling the temperature can allow a surgeon to cool or heat the tissue and/or the organ in contact with the blade and/or fluid loop. This can allow the contacted and surrounding tissue to be cooled to reduce swelling or heated to maintain a patient's temperature.

A further retraction method is shown in Figure 16. The method includes disposing an absorbent layer on the blade of the retractor (step 1000) and setting the blade and the absorbent layer to a retraction position (step 1002). This step is similar to setting the blade and bladder as described above. Fluid is provided to the absorbent layer to moisturize the absorbent layer (step 1004).

Moisturizing the tissue or organ prevents it from drying out when removed from the moist interior of the patient and exposed to the lighting and low humidity air typical in an operating room. The fluid is typically a sterile fluid and can be continuously provided (step 1006) or intermittently provided (step 1008) to the absorbent layer. Also, the temperature of the fluid can be controlled (step 1010) to heat or cool the fluid or keep it at body temperature. The fluid can be provided by a manual or a powered pump. Additionally, the absorbent layer can be soaked in the fluid prior to disposing it on the blade and the absorbent layer can be made of a material that slowly seeps the liquid out at a known rate.

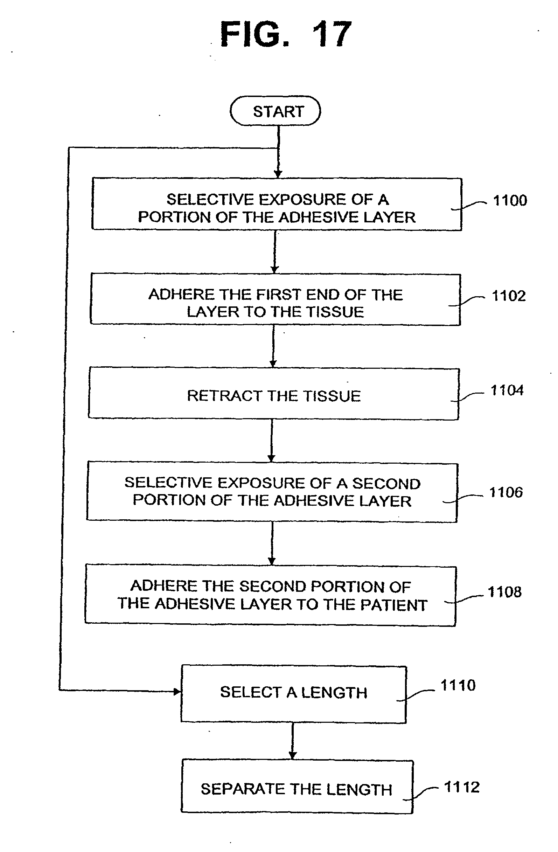

An additional method of retracting tissue, bone or organ using a flexible, disposable retractor is illustrated in Figure17. The steps include selectively exposing a portion of an adhesive layer on a first end of a disposable pad (step 1100). Adhering the first end to the tissue (step 1102) and retracting the tissue (step 1104) by applying tension to the flexible, disposable retractor. A second portion of the adhesive layer on a second end is selectively exposed (step 1106). The selective exposure of the adhesive layer can be performed by removing a segmented portion of a cover layer to expose the adhesive layer. The cover layer can be removed in finite lengths. The surgeon determines the amount of adhesive required for the specific task and can peel back the cover layer to expose only the amount required. The second end is then adhered to the patient, operating table or to the ring member to maintain the retraction of the tissue (step 1108). Once the procedure or portion of the procedure is completed the flexible disposable retractor can be removed at the first end and the second end from the tissue and the patient, respectively.

To assist the surgeon in using the flexible, disposable retractor, the surgeon is able to select a length of the disposable pad (step 1110) and separate the length of disposable pad from a longer length of the disposable pad (step 1112).

While there have been shown, described, and pointed out fundamental novel features of the invention as applied to a preferred embodiment thereof, it will be understood that various omissions, substitutions, and changes in the form and details of the devices illustrated, and in their operation, may be made by those skilled in the art without departing from the spirit and scope of the invention. For example, it is expressly intended that all combinations of those elements and/or steps which perform substantially the same function, in substantially the same way, to achieve the same results are within the scope of the invention. Substitutions of elements from one described embodiment to another are also fully intended and contemplated. It is also to be understood that the drawings are not necessarily drawn to scale, but that they are merely conceptual in nature. It is the intention, therefore, to be limited only as indicated by the scope of the claims appended hereto.

## Claims

1. A disposable cover for a self-retaining retractor having a handle and a blade, the blade having a tissue side that contacts at least one of tissue, a bone and an organ of a patient, and an opposite side, comprising:
a disposable pad having an adhesive side;
an adhesive layer intermittently disposed on the adhesive side of the pad and removably adhering the pad to the blade.

2. The disposable cover of claim 1, wherein the pad only adheres to the tissue side of the blade.

3. The disposable cover of claim 1, wherein the pad adheres to a portion of the tissue side of the blade.

4. The disposable cover of claim 1, wherein the pad further adheres to a portion of the opposite side of the blade.

5. The disposable cover of claim 1, wherein the disposable pad comprises at least one of a conformable polymeric material, a foam, a silastic, acrylic microspheres and a silicone based material.

6. The disposable cover of claim 1, wherein the adhesive is at least one of a coating and a tape.

7. The disposable cover of claim 6, wherein the coating is at least one of Dow™ 4500 and 4600 adhesive solids.

8. The disposable cover of claim 6, wherein the tape is at least one of 3M™ 9877, 1509, 1522, 1524 and 1577 double coated tapes.

9. The disposable cover of claim 1, further comprising a cover layer covering the adhesive layer and selectively removable to expose a portion of the adhesive layer.

10. A disposable cover for a self-retaining retractor having a blade having a tissue side that contacts at least one of tissue, a bone and an organ of a patient, comprising:
a bladder having an adhesive side and elastically expandable under a pressure;
a bladder adhesive layer disposed on the adhesive side of the bladder and removably adhering the bladder to the blade; and
a pump in fluid communication with the bladder selectively supplying the pressure;
wherein the bladder, when pressurized, is padding between the blade and the patient.

11. A disposable cover for a self-retaining retractor having a blade having a tissue side that contacts at least one of tissue, a bone and an organ of a patient, comprising:
a disposable pad removably disposed on the blade;
a bladder disposed within the disposable pad and elastically expandable under a pressure; and
a pump in fluid communication with the bladder selectively supplying the pressure;
wherein the bladder, when pressurized, is additional padding between the blade and the patient.

12. The disposable cover of claim 11, wherein the bladder is a disposable bladder and is permanently disposed within the pad.

13. The disposable cover of claim 11, wherein the bladder is removably disposed within the pad.

14. The disposable cover of claim 11, wherein the pump pressurizes the bladder with at least one of air, fluid and a sterile fluid.

15. The disposable cover of claim 11, wherein the pump is at least one of manual and powered.

16. The disposable cover of claim 11, wherein the pump pressurizes and depressurizes the bladder according to a set interval.

17. The disposable cover of claim 11, wherein the pump randomly pressurizes and depressurizes the bladder.

18. The disposable cover of claim 11, further comprising:
a second disposable pad; and
a second bladder disposed within the second disposable pad and elastically expandable under a pressure;
wherein the pump is in fluid communication with the second bladder.

19. The disposable cover of claim 18, wherein the bladder and the second bladder are pressurized and depressurized concurrently.

20. The disposable cover of claim 18, wherein the bladder and the second bladder are pressurized and depressurized sequentially.

21. The disposable cover of claim 11, further comprising:
a fluid loop disposed within the disposable pad forming a thermal zone;
a pump in fluid communication with the fluid loop and pumping a fluid through the fluid loop; and
a temperature control device controlling a temperature of the fluid and the thermal zone;
wherein the thermal zone regulates a temperature of at least one of the tissue and the organ in contact with the pad.

22. The disposable cover of claim 21, wherein the fluid loop is disposable and is permanently disposed within the pad.

23. The disposable cover of claim 21, wherein the fluid loop is removably disposed within the pad.

24. A disposable cover for a self-retaining retractor having a blade having a tissue side that contacts at least one of tissue, a bone and an organ of a patient, comprising:
a disposable pad removably disposed on the blade;
a bladder disposed within the disposable pad and elastically expandable under a pressure;
a fluid loop disposed within the disposable pad;
a thermal zone comprising the bladder and the fluid loop;
a pump in fluid communication with the bladder selectively supplying the pressure and in fluid communication with the fluid loop and pumping a fluid through the fluid loop; and
a temperature control device controlling a temperature of the fluid and the thermal zone;
wherein the bladder, when pressurized, is additional padding between the blade and the patient and
wherein the thermal zone regulates a temperature of at least one of the tissue and the organ in contact with the pad.

25. The disposable cover of claim 10, wherein the bladder further comprises one or more sub-bladders in fluid communication with the pump.

26. The disposable cover of claim 11, wherein the bladder further comprises one or more sub-bladders in fluid communication with the pump.

27. The disposable cover of claim 25 or 26, wherein the sub-bladders are pressurized and depressurized concurrently.

28. The disposable cover of claim 25 or 26, wherein the sub-bladders are pressurized and depressurized sequentially.

29. A disposable cover for a self-retaining retractor having a blade, comprising:
a disposable pad removably disposed on the blade; and
an absorbent layer disposed within the disposable pad to retain a fluid;
wherein the absorbent layer provides the fluid to the disposable pad.

30. The disposable cover of claim 29, further comprising a pump in fluid communication with the absorbent layer and supplying the fluid to the absorbent layer.

31. The disposable cover of claim 29, further comprising a temperature control device controlling a temperature of the fluid.

32. A flexible, disposable retractor for retracting at least one of tissue and an organ of a patient, comprising:
a disposable pad having an adhesive side, a first end and a second end;
an adhesive layer disposed on the adhesive side of the pad and removably adhering the first end to at least one of the tissue and the organ and removably adhering the second end to the patient;
a cover layer covering the adhesive layer and selectively removable to expose a portion of the adhesive layer;
wherein the disposable pad retracts the at least one of the tissue and the organ to which it is attached.

33. The flexible, disposable retractor of claim 32, having a ring member of a self retaining retraction system mounted to a table, wherein the second end removably adheres to at least one of the ring member and the table.

34. The flexible, disposable retractor of claim 32, further comprising a stiffening member disposed in the retractor.

35. The flexible, disposable retractor of claim 34, wherein the stiffening member had a shape memory.

36. The flexible, disposable retractor of claim 32, wherein the disposable pad has at least one of slits, slots and holes along an edge wherein the slits provide added flexibility to the disposable pad.

37. A surgical retractor for a self-retaining retractor system that includes a hose to conduct one or air and fluid, comprising:
a handle including a distal end;
a blade disposed on the distal end of the handle; and
a channel disposed in the handle permitting the hose to pass through.

38. The surgical retractor of claim 37, wherein the channel is shorter than the handle.

39. The surgical retractor of claim 37, wherein the handle includes a bottom proximate to a patient and the channel is disposed on the bottom of the handle.

40. The surgical retractor of claim 37, wherein the handle furthers comprises:
a proximal connector disposed on a proximal end of the channel removably attaching to the hose; and
a distal connector disposed on a distal end of the channel removably attaching to a line from at least one of a bladder, a fluid loop, and an adsorbent layer;
wherein said channel transports one of the air and the fluid.

41. A ring member for a self-retaining retractor system including a hose to conduct one or air and fluid, comprising a hose raceway disposed on the ring member to support the hose, the ring member having an opening to permit the hose to enter the raceway.

42. The ring member of claim 41, wherein the opening is disposed on an inner perimeter of the ring member.

43. The ring member of claim 41, wherein the opening is disposed on an outer perimeter of the ring member.
